# EUROPÄISCHE PATENTSCHRIFT

(11) **EP 0 820 308 B1**
(45) Veröffentlichungstag und Bekanntmachung des Hinweises auf die Patenterteilung: **01.08.2001**
(21) Anmeldenummer: 96909041.4
(22) Anmeldetag: 12.04.1996
(51) Int. Cl.: A61K 47/48, A61K 41/00, A61K 51/08, A61K 49/00

(54) **KONJUGAT ZUR BEHANDLUNG VON ENTZÜNDUNGSERKRANKUNGEN**
CONJUGATE FOR TREATING INFLAMMATORY DISEASES
CONJUGUE POUR LE TRAITEMENT DE MALADIES INFLAMMATOIRES

(30) Priorität: 13.04.1995 DE 19514088
(43) Veröffentlichungstag der Anmeldung: 28.01.1998
(73) Patentinhaber: Deutsches Krebsforschungszentrum Stiftung des öffentlichen Rechts, 69120 Heidelberg (DE)
(72) Erfinder: SINN, Hannsjörg, D-69118 Wiesloch (DE); SCHRENK, Hans-Herrmann, D-67278 Zeiskamm (DE); MAIER-BORST, Wolfgang, D-69221 Dossenheim (DE); STEHLE, Gerd, D-68305 Mannheim (DE)
(74) Vertreter: Schüssler, Andrea, Dr.
(86) Internationale Anmeldenummer: DE9600644
(87) Internationale Veröffentlichungsnummer: WO9632133

(56) Entgegenhaltungen:
- EP-A- 0 282 057
- EP-A- 0 326 618
- EP-A- 0 352 076
- EP-A- 0 601 183
- EP-A- 0 649 857
- WO-A-87/04351
- WO-A-93/23743
- WO-A-95/12414
- JP-A- 63 267 734
- US-A- 4 767 745
- US-A- 5 171 749
- SCIENCE (WASHINGTON, D. C., 1883-) (1989), 244(4905), 705-7 CODEN: SCIEAS;ISSN: 0036-8075, 1989, XP002012768 MUKHOPADHYAY, AMITABHA ET AL: "Receptor-mediated drug delivery to macrophages in chemotherapy of leishmaniasis"
- CHEMICAL ABSTRACTS, vol. 124, no. 6, 5.Februar 1996 Columbus, Ohio, US; abstract no. 66584, MUKHOPADHYAY, AMITABHA ET AL: "A process for the preparation of compounds for the treatment of diseases affecting macrophages" XP002012769 & IN 173 006 A (COUNCIL OF SCIENTIFIC AND INDUSTRIAL RESEARCH, INDIA)
- INTERNATIONAL JOURNAL OF RADIATION APPLICATIONS AND INSTRUMENTATION PART B: NUCLEAR MEDICINE AND BIOLOGY, Bd. 18, Nr. 6, 1.Januar 1991, Seiten 605-612, XP000236577 THAKUR M L ET AL: "TECHNETIUM-99M-LABELED PROTEINS FOR IMAGING INFLAMMATORY FOCI"
- JOURNAL OF FLUORESCENCE, Bd. 3, Nr. 3, 1.September 1993, Seiten 153-155, XP000563720 TERPETSCHNIG E ET AL: "AN INVESTIGATION OF SQUARAINES AS A NEW CLASS OF FLUOROPHORES WITH LONG-WAVELENGTH EXCITATION AND EMISSION"
- JOURNAL OF PHARMACEUTICAL SCIENCES, Bd. 66, Nr. 3, März 1977, WASHINGTON US, Seiten 414-417, XP002020550 L.E. HARE ET AL.: "ANALYSIS OF SULINDAC AND METABOLITES BY COMBINED ISOTOPE DILUTION-RADIOIMMUNOASSAY."

## Beschreibung

Die Erfindung betrifft die Verwendung eines Konjugats aus einem Zytostatikum als Wirkstoff und Albumin zur Herstellung eines Arzneimittels zur Behandlung und/oder Diagnose von Entzündungserkrankungen.

Bisher werden zur Behandlung von Entzündungserkrankungen Arzneimittel verwendet, die in hohen Dosen, z.T. sogar mehrmals täglich, verabreicht werden. Desweiteren reichern sich diese Arzneimittel in vielen Geweben an. Somit stellen sie eine große Belastung für den Körper dar.

US-A-4, 767, 745 zeigt ein Leukotrien - BSA- Konjugat zur Behandlung von Entzündungserkrankungen.

Aus DE-A-41 22 210 ist ein Konjugat aus einer tumoraktiven Verbindung und einem nicht als körperfremd angesehenen, nativen Protein bekannt. Dieses Konjugat wird zur Behandlung von Tumorerkrankungen verwendet.

Überraschenderweise hat sich nun gezeigt, daß ein solches Konjugat auch zur Behandlung und/oder Diagnose von Entzündungserkrankungen geeignet ist, ohne daß die Nachteile der bisher hierfür verwendeten Arzneimittel auftreten.

Erfindungsgemäß wird somit ein vorstehendes Konjugat zur Herstellung eines Arzneimittels zur Behandlung und/oder Diagnose von Entzündungserkrankungen, wie Psoriasis, verwendet.

Als "Wirkstoff" kommen Zytostatika zum Einsatz. Vertreter dieser sind z.B. Doxorubicin, Daunorubicin, Tetracycline, Antrachinon-2-carbonsäure, Carminsäure, Platinkomplexe und Methotrexat (MTX).

Vorstehende Verbindungen können nachweisbar markiert sein. Dies kann z.B. über eine radioaktive Substanz, wie Jod, erfolgen. Günstig ist es auch, wenn vorstehende Verbindungen eine Säurefunktion, z.B. eine Carboxylgruppe, aufweisen. Damit wird ihre Anbindung an das "Protein" erleichtert.

In einem erfindungsgemäß verwendeten Konjugat können eine oder mehrere vorstehender Verbindungen vorliegen. Bei mehreren, können diese gleich oder verschieden voneinander sein.

Die erfindungsgemäß verwendeten Konjugate umfassen weiter Albumin, insbesondere humanes Serumalbumin (HSA).

Erfindungsgemäß verwendete Konjugate können nach üblichen Verfahren hergestellt werden. Beispielsweise kann die Herstellung nach dem in DE-A-41 22 210 beschriebenen Verfahren erfolgen.

Erfindungsgemäß verwendete Konjugate zeichnen sich dadurch aus, daß sie sich in Zellen und Geweben anreichern, die von entzündlichen Prozessen befallen sind. Zur Behandlung solcher Prozesse reichen daher geringe Dosen dieser Konjugate aus, was die Belastung für den Körper minimiert. Somit eignen sich die erfindungsgemäß verwendeten Konjugate bestens zur Behandlung und/oder Diagnose von Entzündungserkrankungen.

Kurze Beschreibung der Zeichnungen.

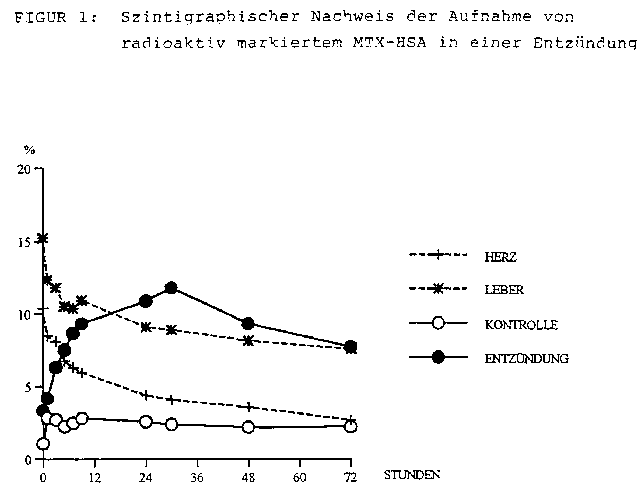

- Fig. 1:: zeigt den szintigraphischen Nachweis der Aufnahme von radioaktiv markiertem MTX-HSA in einer Entzündung.

Das folgende Beispiel erläutert die Erfindung:

### Beispiel: Aufnahme von radioaktiv markiertem MTX-HSA in einer Entzündung am Bein einer Ratte.

In üblicher Weise wurde in einem Bein einer Ratte durch Injektion von Sephadexkügelchen eine Entzündung erzeugt. Anschließend wurde der Ratte das erfindungsgemäße, radioaktiv markierte Konjugat MTX-HSA, verabreicht. MTX-HSA wurde im entzündeten Bein, dem nicht entzündeten Bein (als Kontrolle) sowie der Herz- und der Leberregion szintigraphisch bestimmt.

Wie aus Figur 1 zu sehen ist, reichert sich das erfindungsgemäße Konjugat MTX-HSA im entzündeten Bein an.

## Patentansprüche

1. Verwendung eines Konjugats aus
(a) mindestens einem Zytostatikum als Wirkstoff, und
(b) Albumin
zur Herstellung eines Arzneimittels zur Behandung und/oder Diagnose von Entzündungserkrankungen.

2. Verwendung nach Anspruch 1, wobei aus der Gruppe der Zytostatika mehrere Wirkstoffe vorliegen.

## Claims

1. Use of a conjugate comprising
(a) at least one cytostatic agent as active substance, and
(b) albumin
for the production of a medicament for the treatment and/or diagnosis of inflammatory diseases.

2. Use according to claim 1, wherein several active substances of the group of cytostatic agents are available.

## Revendications

1. Utilisation d'un conjugué formé de :
(a) au moins un agent cytostatique en tant que principe actif et
(b) de l'albumine,
pour la fabrication d'un médicament pour le traitement et/ou le diagnostic de maladies inflammatoires.

2. Utilisation selon la revendication 1, dans laquelle plusieurs principes actifs choisis dans l'ensemble des agents cytostatiques sont présents.
